Europäisches Patentamt
European Patent Office
Office européen des brevets

Publication number: 0 300 082 A2

# EUROPEAN PATENT APPLICATION

Application number: 87112887.2

Int. Cl.4: C12M 1/40

Date of filing: 03.09.87

Priority: 23.07.87 JP 184207/87

Date of publication of application: 25.01.89 Bulletin 89/04

Designated Contracting States: DE FR GB

Applicant: Bridgestone Corporation
10-1, Kyobashi 1-Chome Chuo-Ku
Tokyo 104(JP)

Inventor: Nakajima, Tadashi
No. 10-8, Daita 2-chome
Setagaya-ku Tokyo(JP)
Inventor: Iino, Yasuhiro
No. 3-2-6-304, Ogawahigashi-cho
Kodaira-shi,Tokyo(JP)
Inventor: Kawagoe, Takahiro
No. 1302-57, Aobadai
Tokorozawa-shi Saitama-ken(JP)

Representative: Patentanwälte TER MEER - MÜLLER - STEINMEISTER
Mauerkircherstrasse 45
D-8000 München 80(DE)

## Enzyme electrode.

An enzyme electrode comprises an enzyme immobilized on a polyaniline or a derivative thereof prepared by electrolytic oxidative polymerization or chemical oxydative polymerization using an aqueous acidic solution containing aniline or a derivative thereof. The enzyme electrode is excellent in the current dependency on oxygen concentration and the mediator leaching property and suitable for use in fuel cells, bioreactors, biosensors, etc.

EP 0 300 082 A2

# ENZYME ELECTRODE

## BACKGROUND OF THE INVENTION

### Field of the Invention

The present invention concerns an enzyme electrode suitable as electrode material for fuel cells and bioreactors, electrode for biosensors, etc.

### Description of Prior Art

Technics of utilizing enzymatic reactions have been developed remarkably along with the progress in recent years of biochemistry, and vigorous studies have been made for sensors detecting chemical substances or fuel cells for obtaining electric power by utilizing the enzymatic reactions as one of the field of applying such technics. As an example put to practical use, there can be mentioned a biosensor for the quantitative determination of glucose by utilizing the reaction of oxidizing glucose with glucose oxidase in the following formula (1):

$$C_6H_{12}O_6 + O_2 \xrightarrow{\text{glucose oxidase}} C_6H_{10}O_6 + H_2O_2 \qquad (1)$$

in which $H_2O_2$ formed is determined quantitatively by using a hydrogen peroxide electrode or $O_2$ consumed is determined quantitatively by using an oxygen electrode to detect the amount of glucose. However, there is a problem that no accurate quantitative determination can be obtained by merely utilizing the reaction scheme (1) unless sufficient dissolved oxygen is present. In view of the above, there has recently been made an attempt of conducting electron transfer by using a mediator such as ferrocene, benzoquinone, chloroanil, thionine, etc. and detecting electrical signals. This method has a merit capable of detecting glucose irrespective of the oxygen density in the case of detecting glucose by the following reaction formula (2):

$$C_6H_{12}O_6 + O_2 \xrightarrow[\text{mediator}]{\text{glucose oxidase}} C_6H_{10}O_6 + 2H^+ + 2e^- \qquad (2)$$

However, this method involves a drawback that the mediator substance such as benzoquinone is leached out from the electrode and the sensitivity is lowered gradually.

## SUMMARY OF THE INVENTION

An object of the present invention is to provide an enzyme electrode suitable to biosensors, fuel cells, bioreactors, etc., by using a conductive high molecular substance as the mediator.

The present inventors have made an earnest study for attaining the foregoing object and, as a result, have accomplished the present invention based on the findings that the object can be attained by an enzyme electrode, in which an enzyme is immobilized on a polyaniline or a derivative thereof prepared by electrolytic oxidative polymerization or chemical oxydative polymerization using an aqueous acidic solution containing aniline or a derivative thereof.

The enzyme electrode thus obtained is excellent in the current dependency to the oxygen density and

in the case of measuring the current value of glucose solutions at various concentrations by using glucose oxidase immobilized to the polyaniline or the derivative thereof, for example, it shows satisfactory correspondency between the glucose concentration and the current value up to a relatively high glucose concentration and shows less leaching of polyaniline or the derivative thereof, thus providing excellent mediator leaching property and stable performance for a long period of time.

## BRIEF DESCRIPTION OF THE ACCOMPANYING DRAWINGS

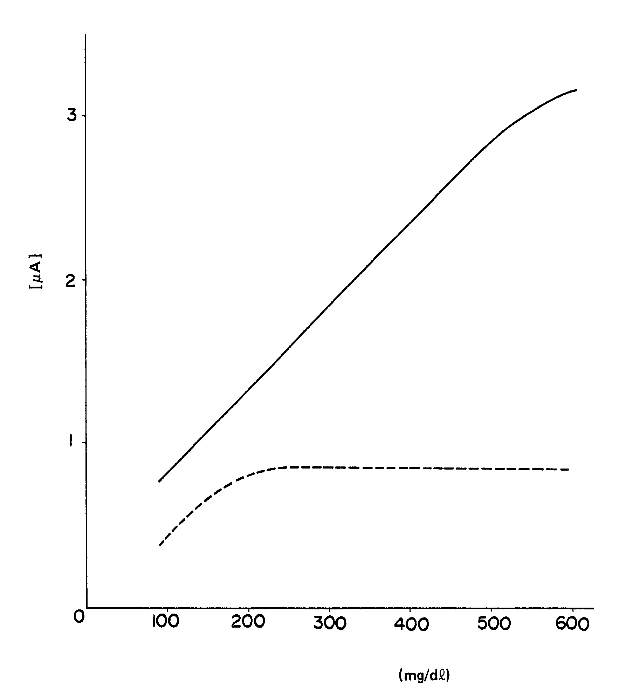

These and other objects, as well as advantageous features of the present invention will become apparent by the following descriptions referring to the accompanying drawing which is a graph showing the relationship between the glucose concentration and the electrical current value in the case of using enzyme electrodes in the example and the comparative example.

## DETAILED DESCRIPTION OF THE INVENTION

This invention will be described more specifically.

The enzyme electrode according to the present invention comprises a polyaniline or a derivative thereof and an enzyme immobilized thereon.

In this case, the polyaniline or the derivative thereof suitably used for the enzyme electrode according to the present invention can be produced by the process of an electrolytic oxidative polymerization in which a polyaniline or a derivative thereof is prepared by electrolytically oxidizing aniline or a derivative thereof on an anode comprising a substrate made of platinum, gold, carbon, stainless steel, etc. in an aqueous acidic solution, or a chemical oxidative polymerization in which a polyaniline or a derivative thereof is prepared by oxidizing aniline or a derivative thereof with an oxidizing agent in an acidic solution. In the present invention, the polyaniline derivative can include, for example,

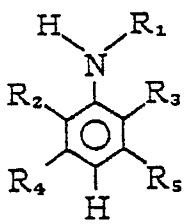

or

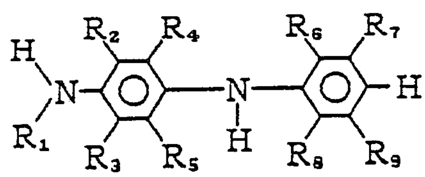

where $R_1$ through $R_9$ respectively represent those groups which are identical or different with each other and selected from : hydrogen atom, aryl group, alkyl group, $NO_2$, $NH_2$, $CH_3$, $SO_2$, CN, $OCH_3$, Cl, F,

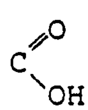 , 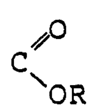 ,

SCN, OCN,

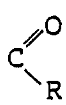 , SO$_2$R, 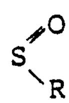 , 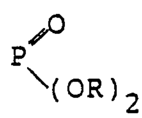 ,

and SR (R represents an aryl or alkyl group). It should be noted that the alkyl group has preferably 1 to 8 carbon atoms and the aryl group has preferably 6 to 12 carbon atoms including substituted or nonsubstituted phenyl group.

In the acidic solution, an aniline concentration ranges preferably from 0.01 to 5 mol/l, more preferably from 0.5 to 3 mol/l, and an acid concentration ranges preferably from 0.02 to 10 mol/l, more preferably from

1 to 6 mol/l. The acidic solution may preferably has a pH of less than 2. As an acid, hydrofluoric acid, hydrochloric acid, nitric acid, perchloric acid, borofluoric acid, sulfuric acid, acetic acid and phosphoric acid are preferably used. More preferred acids are hydrochloric acid, borofluoric acid and acetic acid. These acids are used singly or in combination.

In the case of obtaining the polyaniline or the derivative thereof by the electrolytic oxidative polymerization, the current density may preferably be in the range of 10 μA/cm$^2$ to 20 mA/cm$^2$ and the solution temperature may preferably be in the range of 0 to 40°C, particularly 0 to 20°C. The polymerization time depends on the film thickness required for the use, and usually is in the range of 3 seconds to 6 hours. A cathode may be made of platinum, gold, carbon, stainless steel, etc.

In the case of obtaining the polyaniline or the derivative thereof by the chemical oxidative polymerization (catalytic polymerization process), an oxidizing agent is added in an amount of from 0.01 to 5 mol/l, preferably from 0.5 to 3 mol/l to the acidic solution described above. As the oxidizing agent, known oxidizing agents such as ferric chloride, ammonium persulfate, potassium bichromate and pottassium permanganate are used and, among all, ferric chloride and ammonium persulfate are suitably used. The solution temperature may preferably be in the range of 0 to 40°C, particularly 0 to 20°C. The polymerization time is selected depending on the film thickness required for the use, and usually is in the range of 1 minute to 6 hours.

In the present invention, although the polyaniline or the derivative thereof obtained in this way can be used as it is, the aniline or the derivative thereof applied with various subsequent treatments after the polymerization can be used suitably and, particularly, those applied with reduction and/or neutralization can be used preferably in view of removing background current noises.

In this case, as the method of reduction, an electrolytic method or a method using a reducing agent is employed.

Specifically the electrolytic reduction is carried out by immersing the polyaniline or the derivative thereof as a cathode in an electrolytic solution having a pH of lower than 8, preferably 3 to 6. In this case, platinum, gold, stainless steel, carbon and the like are preferably used as an anode. The electrolytic reduction is preferably carried out at a silver/silver chloride electrode potential of from -0.6 to +0.2 V and at a current density of from 1 to 5 mA/cm$^2$. Depending on the case, it is possible to introduce an oxidizing step at a silver/silver chloride electrode potential of from 0.0 to + 0.7 V, in which a current value of not more than 5 mA is suitable and cyclic voltammetry can be applied at 5 to 50 mV/sec within a range from -0.2 to +0.7 V. In this way, non-biobody ions such as of borofluoric acid and hydrofluoric acid can be exchanged with biobody ions such as of hydrochloric acid and phosphoric acid. As the electrolytic solution for use in the electrolytic reduction or the cyclic voltammetry, borofluoric acid, hydrochloric acid, acetic acid, phosphoric acid, saline water, or known buffer solution using acetic acid, phosphoric acid, phthalic acid, etc. can be used preferably. A suitable range of concentration of the electrolyte is from 5 mol/l to 10 mmol/l and, preferably, from 3 mol/l to 100 mmol/l. Suitable treating temperature is from 0 to 40°C and, more preferably, from 0 to 20°C. From 5 sec to 30 min of time is preferred for electrolysis.

As the reduction treatment using the reducing agent, a method of chemically reducing the polyaniline or the derivative thereof with a reducing agent can be employed. In this case, one or more of hydrogen gas, hydrazine hydrochloride, hydrogen sulfide, sodium sulfite, magnesium, formic acid, etc. may be used as a reducing agent. An aqueous solution containing hydrazine as a reducing agent prepared by adjusting pH to 1 to 10, preferably, 5 to 9 with phosphoric acid, acetic acid, hydrochloric acid, etc is preferred. The concentration of the reducing agent is preferably from 5 mol/l to 50 mmol/l. In the case of using hydrazine, the hydrazine concentration is preferably from 3 mol/l to 50 mmol/l. The acid concentration is preferably from 3 mol/l to 10 mmol/l. Further, the treating time is preferably from one min to 5 hours and the treating temperature is preferably from 0 to 40°C.

As the neutralization method applied as the after-treatment, a method of immersing in or washing by a solution at pH from 3 to 10 and, preferably, from 5 to 9 for one min to 50 hours and, more preferably, from 10 min to 6 hours is employed. The solution may be a known buffer solution. The treating temperature in this case is suitably from 0 to 40°C. A polyaniline synthesized in the aqueous acid solution in the form of an ammonium salt has a pK value of 1 to 3. Accordingly, usual water washing can also be included in the neutralization treatment and, actually, the pK value of the polyaniline is increased up to 3 to 6. The neutralization may be applied simultaneously with the reduction treatment as described above.

In the enzyme electrode according to the present invention, the enzyme to be immobilized on the polyaniline or the derivative thereof as described above is properly selected in view of the substrate specificity and the reaction specificity, etc. depending on the type of the chemical reactions to be taken place and they can include with no particular restrictions, for example, glucose oxidase, alcohol dehydrogenase, urease, glucokinase, peroxidase, cholesterol esterase, lipase, phospholipase, catalase, lactic

acid dehydrogenase, glucoamylase, galactose oxidase, pennicillinase and tyronase.

The method of immobilizing the enzyme on the polyaniline or the derivative thereof can include with no particular restriction, for example, carrier bonding, covalent bonding, ion bonding, adsorption and cross-linking. Among all, a method of dropping an enzyme-containing solution to the polyaniline followed by drying, or a method of forming a Shiff base with glutaraldehyde is preferably used. The amount of the enzyme used based on the polyaniline or the derivative thereof is preferably from 100 to 200,000 units/mg. Suitable treating temperature is from 0 to 30 °C and, more preferably, from 5 to 20 °C and a preferred range for pH is from 3 to 10.

In the enzyme electrode according to the present invention, the polyaniline or the derivative thereof is used as a mediator as described above. In this case, known mediator such as benzoquinone, ferrocene, chloroanil or thionine may be used together with the polyaniline or the derivative thereof, which can provide an effect of extending the measurable range for the substrate concentration.

The enzyme electrode according to the present invention can suitably be used as the electrode for fuel cells, bioreactor cells and biosensors, etc.

In this case, for the constitution of a fuel cell, a method of taking out electrical current by oxidizing various kinds of saccharides, alcohols, methane, ethane, hydrogen, etc. by means of enzymes and microorganisms under the normal temperature operation is suitable. In the case of using the electrode for a biosensor, it is particularly suitable to a blood sugar meter using glucose oxidase or a lactic acid sensor using lactic acid dehydrogenase.

As has been described above, the enzyme electrode according to the present invention is suitably used as electrode for biosensors, fuel cells, bioreactors, etc. and it is excellent when compared with conventional enzyme electrode in view of the current dependency relative to the oxygen concentration and the mediator leaching property.

The present invention will now be explained more specifically referring to an example and a comparative example but the invention is no way restricted only to the following examples.

Example

Using a solution comprising 5 cc of aniline, 15 cc of 42 % borofluoric acid and 30 cc of ion exchanged water as an electrolyte, and two stainless steel plates (SUS 447) sized 2 mm × 20 mm with 250 µm thickness as an anode and a cathode, polyaniline was prepared in an electrolytic polymerization process by supplying a 50 mC of current at a constant current of 20 µA. Then, the thus obtained polyaniline was applied with reduction by cyclic voltammetry in a Clark-Lubs 0.1 mol phosphoric acid buffer solution (pH = 7.4), at a silver/silver halide electrode potential within a range from -200 mV to +500 mV in parallel with neutralization treatment (treating temperature at 25 °C and treating time for 42 minutes).

500 units of glucose oxidase (microorganism origin, manufactured by Oriental Yeast Industry K.K.) was immobilized using 50 µl of a phosphoric acid buffer solution containing 1 % glutaraldehyde to the thus treated polyaniline to obtain an enzyme electrode.

Then, when glucose (manufactured by Wako Junyaku K.K.) was added by a standard addition method to pool serum (Sweetrol N, manufactured by Nissui Seiyaku K.K.) and electric current was measured at a silver/silver chloride electrode potential of +0.5V by using the enzyme electrode, the result as shown by the solid line A in the drawing was obtained, showing that the enzyme electrode of this example had a satisfactory correspondency to the electrical current value up to 500 mg/dl of glucose concentration.

The sensor was stable for the measurement even after 6 weeks (200 cycles of measurement in total).

Comparative Example

A carbon electrode of 2 mm × 20 mm was prepared on a polyethylene film by a screen printing process. The electrode was designed to detect hydrogen peroxide at a silver/silver chloride electrode potential of +0.8 V. Then, 500 units of glucose oxidase (microorganic origin, manufactured by Oriental Yeast Industry K.K.) was immobilized using 50 µl of a phosphoric acid buffer solution containing 1 % glutaraldehyde to the carbon electrode to obtain a comparative enzyme electrode. When glucose (manufactured by Wako Junyaku K.K.) was added by the standard addition method to the pool Serum

(Sweetrol N, manufactured by Nissui Seiyaku K.K.) and the electrical current was measured at a silver silver chloride electrode potential of +0.8 V by using the comparative enzyme electrode, the result as shown by the broken line B in the figure was obtained, in which the enzyme electrode of the comparative example reached its saturation at the glucose concentration of 200 mg/dl.

## Claims

1. An enzyme electrode comprising immobilizing an enzyme on a polyaniline or a derivative thereof prepared by an electrolytic oxidative polymerization or a chemical oxydative polymerization using an aqueous acidic solution containing aniline or a derivative thereof.

2. The enzyme electrode according to claim 1, wherein the electrolytic oxidative polymerization is carried out by using an aqueous acidic solution comprising aniline or a derivative thereof in an aniline amount of from 0.01 to 5 mol/l and an acid in an amount of from 0.02 to 10 mol/l at a current density of 10 $\mu A/cm^2$ to 20 $mA/cm^2$ and at a temperature of 0 to 40 °C.

3. The enzyme electrode according to claim 2, wherein the acid is selected from the group consisting of hydrofluoric acid, hydrochloric acid, nitric acid, perchloric acid, borofluoric acid, sulfuric acid, acetic acid and phosphoric acid.

4. The enzyme electrode according to claim 1, wherein the chemical oxydative polymerization is carried out by using an aqueous acidic solution comprising aniline or a derivative thereof in an aniline amount of from 0.01 to 5 mol/l, an acid in an amount of from 0.02 to 10 mol/l and an oxidizing agent in an amount of from 0.01 to 5 mol/l at a temperature of 0 to 40 °C.

5. The enzyme electrode according to claim 4, wherein the acid is selected from the group consisting of hydrofluoric acid, hydrochloric acid, nitric acid, perchloric acid, borofluoric acid, sulfuric acid, acetic acid and phosphoric acid.

6. The enzyme electrode according to claim 4, wherein the oxidizing agent is selected from the group consisting of ferric chloride, ammonium persulfate, potassium bichromate and potassium permanganate.

7. The enzyme electrode according to claim 1, wherein a reduction treatment is electrically carried out to the polyaniline or the derivative thereof at a silver/silver chloride electrode potential of from -0.6 V to +0.2 V and at a current density of from 1 to 5 $mA/cm^2$ by immersing the polyaniline or the derivative thereof as a cathode in an electrolytic solution having a pH of lower than 8.

8. The enzyme electrode according to claim 1, wherein a cyclic voltammetry is applied to the polyaniline or the derivative thereof at 5 to 50 mV/sec within a range from -0.2 to +0.7 V by immersing the polyaniline or the derivative thereof in an electrolytic solution having a pH of lower than 8.

9. The enzyme electrode according to claim 1, wherein a reduction treatment is chemically carried out to the polyaniline or the derivative thereof by immersing the polyaniline or the derivative thereof in an aqueous solution containing a reducing agent.

10. The enzyme electrode according to claim 1, wherein a neutralization treatment is applied to the polyaniline or the derivative thereof by immersing in or washing by a solution having a pH of 3 to 10.

11. The enzyme electrode according to claim 1, wherein the enzyme is selected from the group consisting of glucose oxidase, alcohol dehydrogenase, urease, glucokinase, peroxidase, cholesterol esterase, lipase, phospholipase, catalase, lactic acid dehydrogenase, glucoamylase, galactose oxidase, pennicillinase and tyronase.

BRIDGESTONE CORPORATION
FAP-443